# EUROPEAN PATENT APPLICATION

(11) **EP 2 505 656 A1**
(43) Date of publication of application: **03.10.2012**
(21) Application number: 12160658.6
(22) Date of filing: 22.03.2012
(51) Int. Cl.: C12P 7/42, C12N 9/00

(54) **Method of producing 3-hydroxypropionic acid using malonic semialdehyde reducing pathway**

(30) Priority: 24.03.2011 KR 20110026531
(71) Applicant: Samsung Electronics Co., Ltd., Suwon-si, Gyeonggi-do, 443-742 (KR)
(72) Inventor: Park, Sung Min, Gyeonggi-do (KR); Koo, Hyun Min, Seoul (KR); Kim, Jae Young, Gyeonggi-do (KR); Yu, Byung Jo, Gyeonggi-do (KR); Cho, Hwa Young, Gyeonggi-do (KR); Park, Young kyoung, Gyeonggi-do (KR); Park, Jae Chan, Gyeonggi-do (KR)
(74) Representative: Grünecker, Kinkeldey, Stockmair & Schwanhäusser

(57) **Abstract**

A method of producing 3-hydroxypropionic acid ("3-HP") with a high yield using a recombinant microorganism having an activity of reducing malonyl CoA into malonic semialdehyde and an activity of reducing malonic semialdehyde into 3-HP and/or an NADPH regeneration activity is provided.

## Description

### CROSS-REFERENCE TO RELATED APPLICATION

This application claims priority to Korean Patent Application No. 10-2011-0026531, filed on March 24, 2011, and all the benefits accruing therefrom under 35 U.S.C. § 119, the disclosure of which is hereby incorporated by reference in its entirety as if fully set forth herein.

### BACKGROUND

### 1. Field

This disclosure relates to a metabolically-modified microorganism and a method of producing the metabolically-modified microorganism, and to a method of producing 3-hydroxypropionic acid ("3-HP") by contacting a suitable carbon substrate with the metabolically-modified microorganism.

### 2. Description of the Related Art

Recently, production of bio-based fuels is becoming imperative, due to the rapid increase in the price of petroleum and serious environmental pollution. Biodiesel, one bio-based fuel, is produced by transesterification of a triglyceride from vegetable oils or animal fats.

Mass production of biodiesel has resulted in large-scale production of glycerol as a byproduct, about 7.7 billion pounds per 1 billion gallons of biodiesel. The production of glycerol has increased very rapidly, and is estimated at about 3.2 billion pounds per year in the United States and 8 billion pounds per year worldwide. As a consequence, the price of glycerol has decreased almost ten-fold over the past 2 years. The market price of crude glycerol was 5 to 15 cents/lb in 2004, but is now reportedly less than 2.5 cents/lb.

In comparison, the price of glucose is currently about 5 cents/lb and is increasing gradually. Should the current trend continue, the continued decrease in the price of glycerol might be considered inevitable.

A microorganism may use glycerol as a carbon source to generate various fermentation products. For example, 3-hydroxypropionic acid (3-HP; C₃H₆O₃. MW 90.08), a compound applicable to various chemical processes, may be produced from a renewable resource such as glycerol.

3-HP may be produced by various chemical synthetic methods or biological methods.

Chemical methods for producing 3-HP may include a method of producing 3-HP from 1,3-propane diol using palladium as a catalyst, a method of producing 3-HP from 3-hydroxypropionaldehyde in the presence of palladium and platinum as catalysts, and a method of producing 3-HP with a selectivity of 91% and a yield of 34% by reaction of acrylic acid with an ion exchange resin (AMBERLYST^{®} 15) as a solid acid catalyst at 100 °C for 40 hours in a highpressure reaction vessel.

The biological production of 3-HP may be performed, e.g., by a photo-heterotrophic microorganism, such as *Chloroflexus aurantiacus.* The microorganism is grown autotrophically or photo-heterotrophically, and thus generates 3-HP as an intermediate product. Other known microorganisms producing 3-HP from glycerol include *Desulfovibrio carbinolicus*, *Desulfovibrio ftuctosovorans*, *Lactobacillus reuteri*, *Pelobacter venetianus*, and *Ilyobacter polytropus.*

### SUMMARY

Conventional biological processes for producing 3-HP are performed by a complicated metabolic pathway. Therefore, it is difficult to effectively control the process, resulting in low production yield and productivity. For this reason, it is necessary to design a 3-HP production pathway that is not present *in vivo.*

Exemplary embodiments provide a method of producing 3-hydroxypropionic acid ("3-HP") using a recombinant microorganism. Unlike the conventional metabolic pathway, the present method may use a "malonic semialdehyde reducing pathway," which reduces acetyl CoA into 3-HP in the order of acetyl CoA, malonyl CoA, malonic semialdehyde and 3-HP, and the activity of re-utilizing NADPH to resolve redox imbalance, thereby obtaining 3-HP with a considerably high yield.

In one aspect, a recombinant microorganism for producing 3-HP, having an activity of reducing malonyl CoA into malonic semialdehyde and an activity of reducing malonic semialdehyde into 3-HP is provided.

Here, the activity of reducing malonyl CoA into malonic semialdehyde may be an activity of a malonyl CoA reductase ("mcr"), and the activity of reducing the malonic semialdehyde into 3-HP may be an activity of a malonic semialdehyde reductase ("msr").

The mcr activity and the msr activity may be exogenous. The mcr activity and the msr activity may all be derived from *M sedula.*

To resolve imbalance between oxidation and reduction in the metabolic process of the microorganism, an NADPH regeneration activity may be further involved.

Here, the NADPH regeneration activity may be a transhydrogenase activity or glyceraldehyde-3-phosphate dehydrogenase activity.

The transhydrogenase activity may be a pyridine nucleotide transhydrogenase *("pntAB")* activity or soluble pyridine nucleotide transhydrogenase ("*udhA*") activity. The *pntAB* activity may be derived from *E. coli.*

The glyeeraldehyde-3-phosphate dehydrogenase activity may be a non-phosphorylating glyceraldehyde-3-phosphate dehydrogenase *("gap*N") activity, which may be derived from *S*. *mutants.*

The genus of available host microorganisms may be one selected from the group consisting of *Zymomonas*, *Escherichia*, *Pseudomonas*, *Alcaligenes*, *Salmonella*, *Shigella*, *Burkholderia*, *Oligotropha*, *Klebsiella*, *Pichia*, *Candida*, *Hansenula*, *Saccharomyces* and *Kluyveromyces,* and preferably, *Escherichia*, *Saccharomyces* and *Kluyveromyces,* which is not limited thereto.

For example, the host microorganism may be *Escherichia coli*, *Kluyveromyces marxianus*, or *Sccharomyces cerevisiae*. In one exemplary embodiment, *Escherichia coli* is used.

A recombinant microorganism in an example may be a microorganism from which *adh*E, *pos*B, *pta*, *ack*, *frd* and/or *ldh*A gene is deleted to effectively perform a desired metabolic pathway.

In another aspect, at least one recombinant vector selected from the group consisting of a vector containing a *mcr-msr* gene, a vector containing a *mcr*-*msr*-*pnt*AB gene, a vector containing a *mcr-msr-gap*N gene, and a vector containing a *mcr*-*msr*-*pnt*AB*-gap*N gene to construct the recombinant microorganism for producing 3-HP is provided.

In still another aspect, a method of producing 3-HP using a microorganism process performing a metabolic pathway is provided, the metabolic pathway including: reducing acetyl CoA into malonyl CoA; reducing the malonyl CoA into malonic semialdehyde; and reducing the malonic semialdehyde into 3-HP.

As described above, the reduction of acetyl CoA into malonyl CoA may be performed by the activity of an acetyl CoA carboxylase, the reduction of malonyl CoA into malonic semialdehyde may be performed by the mcr activity, and the reduction of the malonic semialdehyde into 3-HP may be performed by the msr activity.

Here, a metabolic pathway for providing reducing power using NADPH regeneration activity by a transhydrogenase activity or a glyceraldehyde-3-phosphate dehydrogenase activity may also be involved.

To this end, the method may be performed by culturing a recombinant microorganism having an activity of reducing malonyl CoA into malonic semialdehyde and an activity of reducing malonic semialdehyde into 3-HP and/or a microorganism further having an NADPH regeneration activity in a medium containing a carbon substrate under appropriate conditions.

Such a method increases a malonyl CoA pool and a NADPH and NADH pool in the recombinant microorganism cells.

Available carbon substrates may be, but are not limited to, saccharides, oligosaccharides, polysaccharides, C1 substrates or a combination thereof. For example, the available carbon substrate may be glucose, sucrose, cellulose or glycerol.

According to the method of producing 3-HP, 0.97 g of 3-HP may be obtained per gram of an available carbon source, for example, glucose. This indicates that the maximum yield is 97% (w/w) or more.

As disclosed herein, the recombinant microorganism having the activity of reducing malonyl CoA into malonic semialdehyde, the activity of reducing malonic semialdehyde into 3-HP and the NADPH regeneration activity may be very useful as a strain for producing 3-HP with a high yield.

Therefore, the recombinant microorganism may be useful to improve producibility of 3-HP by combining a malonic semialdehyde reducing pathway through additional manipulation of central metabolic pathways of carbon and by resolving redox imbalance.

### BRIEF DESCRIPTION OF THE DRAWINGS

The above and other aspects of the inventive concept will become more readily apparent by describing in further detail exemplary embodiments thereof which reference to the accompanying drawings, in which:

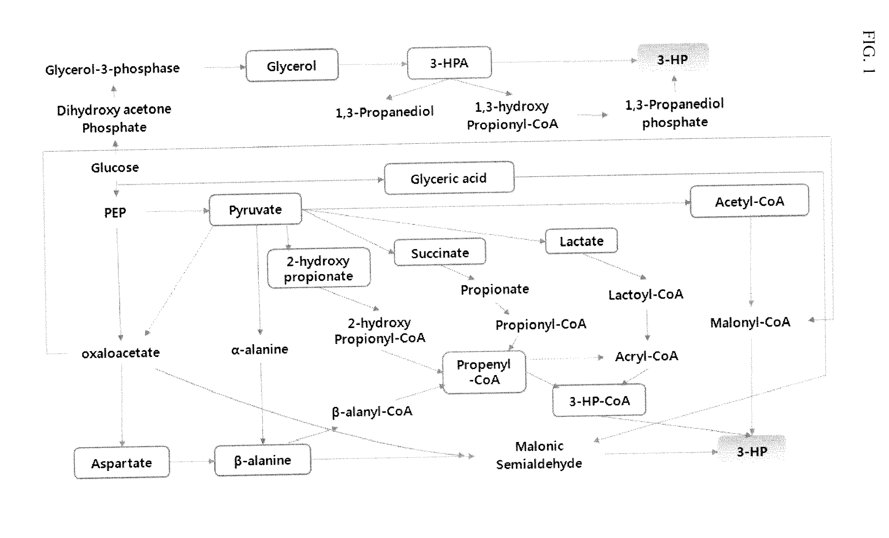

FIG. 1 is a diagram illustrating various metabolic pathways for producing 3-HP;

FIG. 2 is a diagram of a metabolic pathway for producing 3-HP according to an exemplary embodiment of the invention;

FIG. 3A shows a diagram illustrating a wild type metabolic pathway;

FIG. 3B shows a diagram illustrating a genetically engineered metabolic pathway according to an exemplary embodiment of the invention;

FIG. 4 illustrates recombinant vectors constructed according to Example 2;

FIG. 5 presents a graph showing glucose use (upper) and a graph of yield of 3-HP (lower) as a function of time for two exemplary recombinant microorganisms disclosed herein; and

FIG. 6 presents a graph showing glucose use (upper) and a graph a yield of 3-HP (lower) as a function of time for three exemplary recombinant microorganisms.

### DETAILED DESCRIPTION

Definitions of the terms used herein are as follows.

The term "nucleic acid" is well known in the art. "Nucleic acid" as used herein is a molecule of DNA, RNA, or a derivative or analog thereof, comprising nucleobases. For example, the nucleobases include naturally occurring or modified purine or pyrimidine bases. For example in DNA, the naturally occurring nucleobases are adenine "A," guanine "G," thymine "T," and cytosine "C" and in RNA the naturally occurring nucleobases are A, G, urasil "U", and C.

A "polypeptide" is a single linear chain of amino acids bonded together by peptide bonds. A "polypeptide" also includes a polypeptide modified after translation. The terms "polypeptide" and "protein" herein can encompass a single polypeptide chain orcomplexes of two or more polypeptide chains.

The term "protein" includes a fragment, an analog, or a derivative of a given protein having essentially the same biological activity or function as the given protein.

The term "enzymatic activity" refers to a function of stimulating a chemical reaction of a different material without destruction or modification until the end of the reaction.

The expression "encoded by" or "coding for" is used to explain that a sequence of a nucleic acid codes for a polypeptide sequence. Here, a polypeptide sequence encoded by a nucleic acid sequence includes an amino acid sequence composed of at least 3 to 5 amino acids, at least 8 to 10 amino acids, or 15 to 20 amino acids.

The term "gene" refers to a nucleotide sequence of a nucleic acid molecule (chromosome, plasmid, etc.) related to a genetic function. A gene is a genetic unit of an organism including, for example, a polynucleotide sequence occupying a specific physical location ("genetic locus") in the genome of the organism (for example, a DNA sequence of a mammal). A gene may code for an expression product such as a polypeptide or a polynucleotide. Generally, a gene includes a coding sequence, such as a polypeptide coding sequence, and may also include non-coding sequences, such as a promoter sequence, a polyadenylated sequence, or a transcription control sequence (e.g., an enhancer sequence). Genes of various eukaryotic organisms have "exons (coding sequences)" between which an intron "non-coding sequence)" is interposed.

The terms "transformation" and "transfection" refer to the process by which an exogenous DNA is introduced into a host cell. The term "transfected cell" refers to a cell having exogenous DNA introduced into the cell. When DNA is introduced into the cell, the nucleic acid may be inserted into a chromosome or replicated as extrachromosomal material.

The term "vector" refers to an arbitrary nucleic acid including a competent nucleotide sequence, which is inserted into a host cell to be recombined with the genome of a host cell or to autonomously replicate within the host cell as an episome. Such a vector may be a linear nucleic acid, a plasmid, a cosmid, an RNA vector, or a viral vector.

The term "host cell" includes an individual cell or a cell culture, which serves to receive and harbor an arbitrary recombinant vector(s) or isolated polynucleotide. The host cell may be a descendant of a single host cell, and the descendant may not be completely the same as a parent cell due to natural, accidental or artificial mutagenesis and/or variation (in an aspect of its phenotype or total DNA complement). A host cell may be transfected, transformed, or infected by a recombinant vector or polynucleotide *in vivo* or *in vitro*. A host cell including a recombinant vector is referred to interchangeably herein as a recombinant host cell, a recombinant cell, or a recombinant microorganism.

The term "conditions for an enzyme reaction" refers to the arbitrary conditions (e.g., temperature, pH, a non-inhibitory material) providing an environment allowing an enzyme to function catalytically. The conditions for the enzyme reaction may be *in vitro* or *in vivo* conditions, such as conditions in a test tube or in a cell.

The term "metabolically engineered" or "metabolic engineering" involves rational pathway design and assembly of biosynthetic genes, genes associated with operons, and control elements of such polynucleotides, to produce or increase production of a desired metabolite from a microorganism. "Metabolically engineered" may further include optimization of metabolic flux by regulation and optimization of transcription, translation, protein stability, and/or protein functionality using genetic engineering and suitable culture conditions including reduction, disruption, or knocking out of a competing metabolic pathway that competes for an intermediate leading to a desired pathway. A gene can be exogenous to the host microorganism, either by virtue of being foreign to the host, or by being modified by mutagenesis, recombination, and/or association with an exogenous expression control sequence in the host cell. In one aspect, when a gene is xenogenetic to the host organism, the gene can be codon-optimized for expression in the host organism.

The term "substrate" refers to any substance or compound that is converted or meant to be converted into another compound by action of an enzyme. The term includes not only a single compound, but also combinations of compounds, such as solutions, mixtures, and other materials, which contain at least one substrate, or derivative thereof. Substrates include suitable carbon sources ordinarily used by microorganisms. Further, the term "substrate" encompasses not only compounds that provide a carbon source suitable for use as a starting material, such as any biomass-derived sugar, but also intermediate and end product metabolites used in a pathway of a metabolically engineered microorganism as described herein.

The terms "function" and "functionality" refer to a biological or enzymatic function.

The expression "increased", or "increasing", amount of a given product or molecule (e.g., from a wide-range of chemicals, biofuels or an intermediate thereof) is used to denote that a recombinant microorganism can generate the given product or molecule in a larger amount than a control microorganism such as a non-modified or differently-modified microorganism.

The term "obtained from" or "derived from" when used in reference to a sample or a polynucleotide or polypeptide means that the sample, such as a nucleotide extract or polypeptide extract, or the polynucleotide or polypeptide is isolated or induced from a specific source such as a predetermined organism, typically a microorganism.

The term "recombinant microorganism" referes to a microorganism that typically includes at least one exogenous nucleotide sequence in a plasmid or vector.

The terms "approximately" and "about" are interchangeably used herein and indicate an amount, level, value, number, frequency, percent, dimension, size, weight or length changed by 30, 25, 20, 25, 10, 9, 8, 7, 6, 5, 4, 3, 2 or 1% from a reference amount, level, value, number, frequency, percent, dimension, size, weight or length.

Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable.

It will be further understood that the terms "comprises" and/or "comprising" when used in this specification, specify the presence of steps or elements, or groups thereof, but do not preclude the presence or addition of one or more other steps or elements, or groups thereof.

Unless otherwise defined, all terms used herein have the same meaning as commonly understood by those skilled in the art. In addition, methods or samples are described in the specification, but methods or samples similar to or the same as those described above are also included in the scope of the invention. The contents of all publications described are herein incorporated by reference.

In one aspect of the invention, a method of producing 3-HP using a recombinant microorganism is provided. Genes in a "malonic semialdehyde reducing pathway" for sequentially reducing acetyl CoA into 3-HP in the order of acetyl CoA, malonyl CoA, malonic semialdehyde, and 3-HP are introduced into a host to obtain the recombinant microorganism. Culturing the recombinant microorganism disclosed herein permits efficient production of 3-HP in high yield via the malonic semialdehyde reducing pathway.

By using standard cloning techniques and conventional methods known by those skilled in the art, the recombinant microorganism may be obtained by inserting a gene of interest into a vector to transform a host microorganism, and culturing the transformed recombinant microorganism. Therefore, a method of producing 3-HP, method of expressing a related enzyme, and a recombinant microorganism are provided by the invention.

In an embodiment, a method of producing 3-HP using a microorganism is provided.

3-HP is a weak three carbon non-chiral organic acid having a pKa of 4.51 at 25 °C, which is an isomer of 2-hydroxypropionic acid (lactic acid). Furthermore, 3-HP is an amorphous, viscous yellow liquid, having a specific gravity of 1.25 and a refractive index of 1.45.

3-HP is very soluble in water, and tthe calcium salt of 3-HP is 100 times more soluble in water than citric acid or malic acid. Therefore, 3-HP is useful for preventing scale, for example, in a boiler or in industrial equipment. In addition, 3-HP is a critical synthetic intermediate in some chemical processes. Particularly, 3-HP is significant for production of some chemicals and polymers, including for example production of malic acid by oxidation, production of a biodegradable polyester known as poly(3-hydroxypropionic acid) by esterification with alcohol, and production of 1,3-propanediol by reduction.

3-HP is a critical synthetic intermediate in various chemical processes, and is used as a source to generate 1,3-propanediol (C₃H₈O₂ - MW 76.09), acrylic acid (C₃H₄O₂ - MW 72.06), methyl acrylate (C₄H₆O₂ - MW 86.09), acrylamide (C₃H₅NO - MW 71.08), ethyl 3-hydroxypropionic acid (C₅H₁₀O₃ - MW 118.13), malonic acid (C₃H₄O₄ - MW 104.06), propiolactone (C₃H₄O₂ - MW 72.06), or acrylonitrile (C₃H₄N - MW 53.06).

Biosynthetic Pathway of 3-HP

In one embodiment, a biosynthetic pathway for 3-HP which is metabolically-engineered using a pathway of producing an intrinsic energy of an organism is included.

A more host-friendly biofuel system using an intrinsic metabolite of an organism is provided by the biosynthetic pathway for producing a biofuel.

The term "biosynthetic pathway," also referred to as a "metabolic pathway," is a set of anabolic or catabolic biochemical reactions for transmuting one chemical species into another. Gene products belong to the same "metabolic pathway" if they, in parallel or in series, act on the same substrate, produce the same product, or act on or produce a metabolic intermediate (i.e., metabolite) between the same substrate and metabolite end product.

A biosynthetic pathway for 3-HP uses a carbon source as a substrate. For example, the carbon source may be selected from the group consisting of a monosaccharide, an oligosaccharide, a polysaccharide, a C1 substrate, and a mixture thereof. Particularly, the carbon source may include, but is not limited to, alginate, agar, carrageenan, fucoidan, pectin, gluconate, mannuronate, mannitol, rixose, cellulose, hemicellulose, glycerol, xylitol, glucose, sucrose, mannose, galactose, xylose, xylan, mannan, arabinan, arabinose, glucuronate, galacturonate (such as di- or tri-galacturonate), and rhamnose. In an exemplary embodiment, the carbon source is glucose, sucrose, glycerol, and/or cellulose.

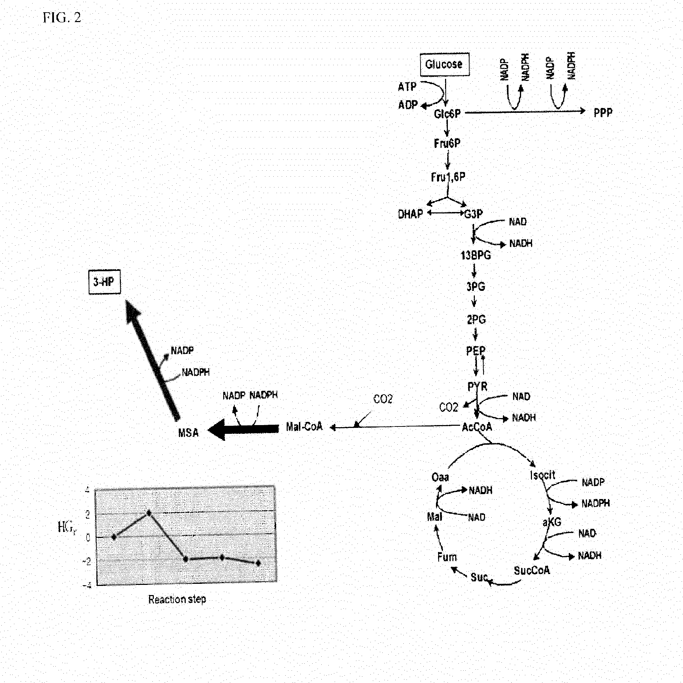

To describe the biosynthetic pathway for producing 3-HP, as an exemplary embodiment, a "biosynthetic pathway to 3-HP" using glucose as the carbon source is illustrated in FIG. 2.

Referring to FIG. 2, significant metabolic pathways for producing energy are the same as the common pathways well known in the art, for example, glycolysis , converting glucose into pyruvate, and the tricarboxylic acid ("TCA") cycle, oxidizing pyruvate generated in glycolysis into CO₂ and H₂O via acetyl CoA.

Glycolysis

Glucose is a major carbon source for many living organisms. Degradation of glucose in cells starts with glycolysis. Glycolysis, also known as the Embden-Meyerhof-Parnas ("EMP") pathway., is a series of reactions which convert glucose into pyruvate and incidentally generate 2 moles of ATP and 2 moles of NADH per mole of glucose. In the overall process of the glycolysis, the 6 carbon ring of glucose becomes unstable due to energy obtained from two ATP molecules, and then is degraded into two molecules of triose ("C3"). Afterwards, the triose ("C3") becomes stable by emitting energy, thereby obtaining pyruvate. Glycolysis occurs in the cell cytoplasm. In the first step of glycolysis, one ATP is used to generate glucose 6-phosphate by phosphorylation of glucose catalyzed by hexokinase. The glucose 6-phosphate is converted into fructose 6-phosphate in a reaction catalyzed by phosphoglucose isomerase. The fructose 6-phosphate is converted into fructose 1,6-diphosphate by phosphorylation using ATP in a reaction catalyzed by phosphofructokinase. The fructose 1,6-diphosphate is then converted into dihydroxyacetone phosphate ("DHAP") and glyceraldehyde-3-phosphate ("GA-3P") by a reaction catalyzed by aldolase, thereby degrading one molecule of hexose ("C6") into two molecules of triose ("C3"). DHAP and GA-3P are in equilibrium. The glyceraldehyde 3-phosphate can continue directly in the glycolytic pathway. However, DHAP must be converted to glyceraldehyde 3-phosphate in order to continue in the pathway. This isomerization is catalyzed by triose phosphate isomerase. As GA-3P is consumed, DHAP is continuously converted into GA-3P. Inorganic phosphoric acid ("Pi") is added to GA-3P in a reaction catalyzed by glyceraldehyde-3-phosphate dehydrogenase to produce 1,3-diphosphoglycerate. The 1,3-diphosphoglycerate emits one phosphate group to generate 3-phosphoglycerate and ATP generated from ADP in a reaction catalyzed by phosphoglycerate kinase to generate 3-phosphoglycerate. The 3-phosphoglycerate is catalytically converted into 2-phosphoglycerate by phosphoglyceromutase, and then the 2-phosphoglycerate is dehydrogenated in a reaction catalyzed by enolase to produce phosphoenolpyruvate ("PEP"). The PEP is converted to pyruvate with production of ATP in a dephosphorylation reaction catalyzed by pyruvate kinase.

TCA Cycle

The tricarboxylic acid ("TCA") cycle, also known as the Krebs cycle, is a process in which the pyruvate generated by glycolysis can be converted into carbon dioxide and hydrogen. Via the TCA cycle, the pyruvate generated by the glycolysis is completely oxidized in the mitochondria, and reducing power is delivered to NAD⁺, with generation of CO₂. Major roles of the TCA cycle are (1) generation of electrons (NADH) required for biosynthesis and the electron transport chain, (2) supply of carbon-based backbone material for amino acid synthesis, and (3) generation of energy.

The pyruvate generated by glycolysis is catalytically converted into acetyl CoA by pyruvate dehydrogenase. This acylation step serves to connect glycolysis with the TCA cycle.

Pyruvate + CoA + NAD⁺ → Acetyl CoA + CO₂ + NADH + H⁺

The acetyl CoA is delivered into the mitochondria through a membrane, and energy required for the delivery of the acetyl CoA is obtained by converting two NADH generated by glycolysis into two FADHs. Aacetyl CoA is an intermediate, which plays a major role in metabolism of amino acids and fatty acids. The TCA cycle is a final common pathway to oxidize fuel molecules such as amino acids, fatty acids and carbohydrates.

The sequence of the TCA cycle will be described in detail: Citrate ("C6") is generated in a condensation reaction between acetyl CoA ("C2") and oxaloacetate ("C4"), and then converted into isocitrate ("C6"). Subsequently, the isocitrate ("C6") is converted into α-ketoglutarate C"C5"), with generation of one CO₂ =and one NADH. The a-ketoglutarate is converted into succinyl CoA ("C4") through decarboxylation and oxidation, with generation of another CO₂ and one NADH. The succinyl CoA ("C4") is converted into succinate ("C4"), with generation of a GTP, witha high energy phosphate bond. The succinate is oxidized into fumarate ("C4"), with generation of one molecule of 1,5-dihydro-flavin adenine dinucleotide (FADH₂). The last two steps of the TCA cycle include generation of malate ("C4") through hydration of fumarate, and generation of oxaloacetate ("C4") through oxidation of malate.

In the four oxidation-reduction reactions occurring in the TCA cycle, three pairs of electrons are transferred to NAD⁺, and one pair of electrons are transferred to flavin adenine dinucleotide (FAD). The reduced electron carriers will be oxidized in the electron transport chain, generating 9 moles of ATPs. In addition, one high energy phosphate bond is directly formed when succinyl CoA is converted into succinate. Therefore, 10 moles of high energy phosphate bonds are generated per one acetyl CoA completely oxidized into H₂O and CO₂.

The glycolysis and TCA cycle are generally known in the art. A method of producing 3-HP according to one embodiment uses a pathway in which acetyl CoA is converted into malonyl CoA and reduced into malonic semialdehyde, which is subsequently converted to 3-HP. That is, the "atonic semialdehyde reduction pathway" is used.

Therefore, the method of producing 3-HP disclosed herein includes the following steps: reducing acetyl CoA into malonyl CoA, reducing malonyl CoA into malonic semialdehyde; and reducing malonic semialdehyde into 3-HP.

First, the 3-HP production pathway disclosed herein includes reducing acetyl CoA into malonyl CoA, as shown in the following reaction equation.

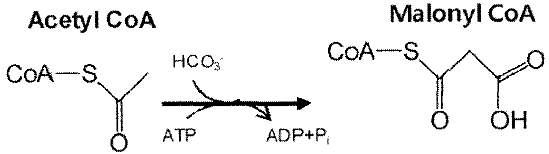

The acetyl CoA is converted into malonyl CoA through carboxylation. For example, the carboxylation is an irreversible committed step, which may be catalyzed by an acetyl CoA carboxylase ("ACC"). This enzyme is very similar to a pyruvate carboxylase. These enzymes contain biotin as a prosthetic group, and their reaction mechanisms are similar to each other. Carbon dioxide ("CO₂") in the form of bicarbonate forms carbonyl phosphate, which is an activated form of CO₂, by attacking a phosphate group of ATP. Biotin reacts with the carbonyl phosphate to form N-carboxybiotin with release of phosphate (Pᵢ), and then the carboxyl group is transferred to acetyl CoA, thereby generating malonyl CoA.

Prokaryotes and plants have multi-subunit ACCs composed of several polypeptides encoded by distinct genes. To increase the endogenous activity of acetyl CoA carboxylase in E. coli, transformation of an E. coli host with an overexpression vector for *E. coli*-derived accABCD genes encoding the acetyl CoA carboxylase may be performed.

In this step, the malonyl CoA pool is increased in the recombinant microorganism.

The 3-HP production pathway also includes the steps of f reducing the malonyl CoA into malonic semialdehyde and then reducing the malonic semialdehde to 3_HP, as shown in the reaction scheme below.

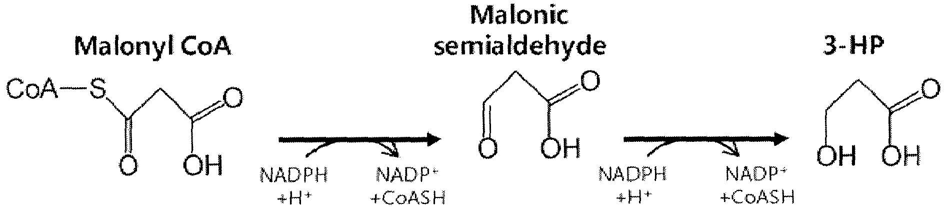

Here, the reduction of malonyl CoA uses an enzyme activity for reducing malonyl CoA and reduction of malonic semialdehyde uses an enzyme activity for reducing malonic semialdehyde). To this end, these enzyme activities may be expressed in and/or obtained from a recombinant microorganism metabolically modified to express or overexpress these enzyme activities.

The activity for reducing malonyl CoA into malonic semialdehyde may be a malonyl CoA reductase (malonate semialdehyde-forming) (mcr) activity. The malonic semialdehyde, which is the metabolic product obtained from the reduction, may be used to produce 3-HP. The activity for reducing the malonic semialdehyde into 3-hydroxypropionic acid ("3-HP") may be a malonic semialdehyde reductase (msr) activity.

For the 3-HP biosynthetic pathway disclosed herein, further metabolic modification of the host organism may be performed to express or overexpress an enzyme having an NADPH regeneration activity to resolve redox imbalance.

Reducing power plays a major role in a living cell along with energy. Hydrogen atoms generating reducing power are generally delivered by nucleotide derivatives, which include nicotinamide adenine dinucleotide ("NAD⁺") and nicotinamide adenine dinucleotidephosphate ("NADP⁺"). nicotinamide adenine dinucleotide (reduced form) ("NADH") to which the hydrogen is attached plays two roles in providing reducing power into the living cell and generating ATP. That is, NADH provides hydrogen to biosynthesis reaction such as CO₂ fixation by autotrophic organisms (plants and photosynthetic bacteria). Electrons (or hydrogen atoms) delivered by NADH sequentially go through several intermediate compounds and are finally delivered to oxygen in the electron transport chain. A maximum of 3 ATP molecules are generated by energy emitted in the above-mentioned electron transport chain.

The activity for regenerating NADPH to increase levels of NADPH and NADH may be a pyridine nucleotide transhydrogenase activity, for example, pntAB from E. coli) and/or a glyceraldehyde 3-HP dehydrogenase activity (for example, gapN from S. mutans).

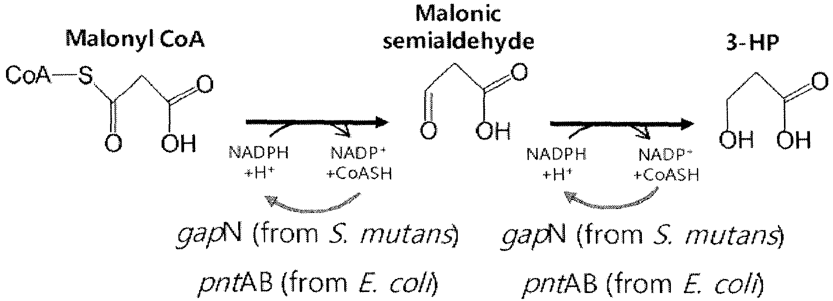

For the 3-HP biosynthetic pathway disclosed herein, various enzymes are used. In addition to the known enzymes used in glycolysis and the TCA cycle, additional exogenous enzymes are used, for example, an enzyme used in the malonic semialdehyde reducing pathway or an NADPH regeneration enzyme. These various enzymes produce various metabolic products mentioned above.

A suitable polynucleotide encoding a desired enzyme, or one of its subunits, may be derived from a certain biological source providing the same, and its homologues may be determined with reference to various databases known in the art.

The native DNA sequence(s) encoding a biosynthetic enzyme described above are referenced herein merely to illustrate an exemplary embodiment. The invention includes DNA molecules of any sequence that encode the amino acid sequence of a polypeptides of an enzyme used in the method. In similar fashion, a polypeptide may typically tolerate at least one amino acid substitution, deletion, or insertion in its amino acid sequence without loss or significant loss of a desired activity. Modified polypeptides or variant polypeptides having the enzymatic anabolic or catabolic activity of the wild-type polypeptide are contemplated by the invention. Furthermore, the amino acid sequences encoded by the DNA sequences shown herein merely illustrate and exemplary embodiment.

Sequences of the genes and polypeptides/enzymes mentioned above may be easily determined with reference to an available database on the internet, for example, the E. coli protein database (EcoProDB), KAIST, 373-1 Guseong-dong, Yuseong-gu Daejeon 305-701, Republic of Korea In addition, these amino acid and nucleic acid sequences may be easily compared in identity using an algorithm (e.g., BLAST) generally used in the art.

Recombinant Microorganism

A metabolically engineered microorganism (recombinant microorganism) including a biochemical pathway to produce 3-HP from a suitable substrate is provided.

One embodiment of the metabolically-engineered microorganism includes at least one recombinant polynucleotide inside or outside the genome of the microorganism. Such a microorganism has a reduction in expression of an endogenous gene, disruption in expression of an endogenous gene, or a knockout of an endogenous gene, and/or the introduction of a exogenous polynucleotide.

In an embodiment, a recombinant microorganism for generating 3-HP having an activity for reducing malonyl CoA into malonic semialdehyde and an activity for reducing malonic semialdehyde into 3-HP is provided.

The activity for reducing malonyl CoA into malonic semialdehyde is a malonyl CoA reductase (malonate semialdehyde-forming) (mcr) activity. In an embodiment the mcr activity may be derived from, but is not limited to, *Acidianus brierleyi*, *Candida rugosa*, *Candida tropicalis*, *Chloroflexus aurantiacus*, *Clostridium propionicum*, *Clostridium kluyveri*, *Comamonas acidororans*, *Escherichia coli*, *Chloroflexus aurantiacus*, *Candida rugosa*, *Megasphaera elsdenii*, *Pseudomonas fluorescens*, *Pseudomonas oleovorans*, *Ralstonia eutropha*, *Rhodobacter capsulates*, *Rhodobacter sphaeroides*, *Rhodospirillum rubrum*, *Saccharomyces cervisiae, Salmonella enterica*, *Sulfolobus metacillus* or *Metallosphaera sedula*. The activity of reducing malonic semialdehyde into 3-HP may be a malonic semialdehyde reductase (msr) activity. In an embodiment, the msr activity may be derived from *M sedula, Sulfolubus tokodaii*, *Roseiflexus sp. RS-1*, *Roseiflexus castenholzii,* or *Chloroflexus aggregans*.

The mer and msr genes may be variants thereof which respectively encode a malonyl CoA reductase and a malonic semialdehyde reductase having substantially the same or superior reduction activities as compared to the wildtype enzymes. In an exemplary embodiment, the mcr gene encodes an enzyme that may be, but is not limited to, EC 1.2.1.75, and the msr gene may be, but is not limited to, Msed_1993, a 3-hydroxyacyl-CoA dehydrogenase (see United States National Center for Biotechnology Information Gene ID: 5103380; Kochelkorn and Fuchs, Journal of Bacteriology, 2009, 191(20), p.6352-6362).

The recombinant microorganism may further have an NADPH regeneration activity to resolve the redox imbalance.

The NADPH regeneration activity may be a pyridine nucleotide transhydrogenase activity and/or a glyceraldehyde-3-phosphate dehydrogenase activity.

Therefore, a recombinant microorganism, for example, may further have a pyridine nucleotide transhydrogenase ("*pnt*AB") activity and/or a glyceraldehyde-3-phosphate dehydrogenase ("*gap*N") activity. The *pnt*AB activity may be derived from, but is not limited to, *E. coli*, and the *gapN* activity may be derived from, but is not limited to, *E. coli*, *S. cerevisiae*, *Methanococcus maripaludis*, *B. subtilis or S.*

The NADPH and/or NADH pool is increased in a recombinant microorganism containing the above-mentioned genes.

That is, in one embodiment, when a NADPH regeneration activity is further provided in the recombinant microorganism, the NADPH and/or NADH pool is increased, and thus the redox imbalance is resolved. Therefore, 3-HP may be obtained with a significantly higher yield.

The activity for reducing malonyl CoA into malonic semialdehyde, the activity for reducing malonic semialdehyde into 3-HP, and the NADPH regeneration activity (e.g., the *pntAB* activity and/or the gapN activity) may be introduced to a microorganism by a known method in the art. For example, the method may include manufacturing a vector including a gene for expressing the desired activity and transforming a microorganism with the recombinant vector.

Recombinant Vector

A vector refers to a DNA construct comprising a DNA sequence operably linked to a suitable control sequence capable of expressing the DNA in a suitable host microorganism.

The vector may be a plasmid, a phage particle, or a potential simple genomic insert. When a suitable host microorganism is transformed with the vector, the vector can either replicate itself and operate irrespective of the host genome or, in some cases, the vector recombines with the host genome. A plasmid is currently most often used as a vector, thus a "plasmid" herein is interchangeably used to mean a vector. However, any of the vectors well known in the art or various types of vectors having the same function as those well known in the art may be used.

As known to those skilled in the art, to increase the expression level of a gene introduced into a host cell, the gene should be operably linked to expression control sequences for control of transcription and translation, which function in the selected expression host. For example, the expression control sequences and the gene are included in one expression vector together with a selection marker and a replication origin. When the expression host is a eukaryotic cell, the expression vector should further include an expression marker useful in the eukaryotic expression host.

The term "operably linked" indicates that elements are arranged to permit the general functions of the elements. Therefore, a certain promoter operably linked to a coding sequence (e.g., a sequence coding for a polypeptide of interest) may enable expression of the coding sequence in the presence of a control protein and a suitable enzyme. In some cases, such a promoter is not necessarily adjacent to the coding sequence as long as the specific control element can direct the expression of the coding sequence.

The term "expression control sequence" refers to a DNA sequence necessary for the expression of an operably linked coding sequence in a specific host organism. An expression control sequence includes a promoter for performing transcription, an arbitrary operator sequence for controlling transcription, a sequence encoding a ribosome binding site, and sequences for controlling termination of transcription and translation. In addition to transcription initiation and control sites, the expression vector may also include a transcription termination sequence, and a ribosome-binding site for transcription in a transcription region as well as. For example, a polyadenylated signal may be included in an expression construct for polyadenylation suitable for a transcript. It is not considered that a property of the polyadenylated signal is significant to successful performance, and thus an arbitrary sequence of the polyadenylated signal may be used. Further, those skilled in the art may see various control sequences useful to an expression vector. The vector may also include a selection marker which can select a minor group of cells containing a recombinant vector product. The marker may be contained in the same vector as that containing a cloned gene sequence disclosed herein, or be present on a separate vector. A selection marker is a nucleic acid sequence which grants a traceable characteristic to a cell in order to easily identify, isolate, or select a cell having the selection marker from a cell not containing the selection marker during expression. An arbitrary selection marker known in the art may be used as a selection marker in the nucleic acid. In an embodiment, the selection marker is an antibiotic resistance gene.

In an example, to express a desired DNA sequence (e.g., a sequence coding for an enzyme), any one among various expression control sequences known in the art may be used in the vector. Examples of useful expression control sequences may include an SV40 promoter or the early and late promoters of adenovirus, the lac operon system, the trp operon system, the TAC or TRC system, T3 and T7 promoters, the major operator and promoter domain of phage lambda, the control region of fd coat protein, 3-phosphoglyceratekinase or other glycolytic enzymes, the promoters of a phosphatase, for example, Pho5, the promoter of the yeast alpha-mating system and the sequences of a construct known for controlling the expression of genes of prokaryotes, eukaryotes, or viruses thereof, and their various combinations.

In an exemplary embodiment, in forming a recombinant vector, various vectors such as a plasmid vector, a bacteriophage vector, a cosmid vector, or a yeast artificial chromosome ("YAC") vector may be used. In one embodiment, a plasmid vector is used.

A typical plasmid vector that can be used for these purposes has (a) an origin of replication so that it leads to effective replication to result in several hundred copies of the plasmid vector per each host cell, (b) an antibiotic-resistance gene so that a host cell transformed with the plasmid vector can be selected, and (c) a sequence comprising a restriction enzyme site where an exogenous DNA fragment can be inserted. Even in the absence of a suitable restriction enzyme site, a vector and the exogenous DNA can easily be ligated by using a synthetic oligonucleotide adaptor or a linker according to conventional methods known in the art.

Conventionally, the DNA sequence and vector are digested with at least one restriction enzyme and then ligated with each other, to connect the DNA sequence to be expressed to the vector. Digestion by a restriction enzyme and ligation are well known by those skilled in the art.

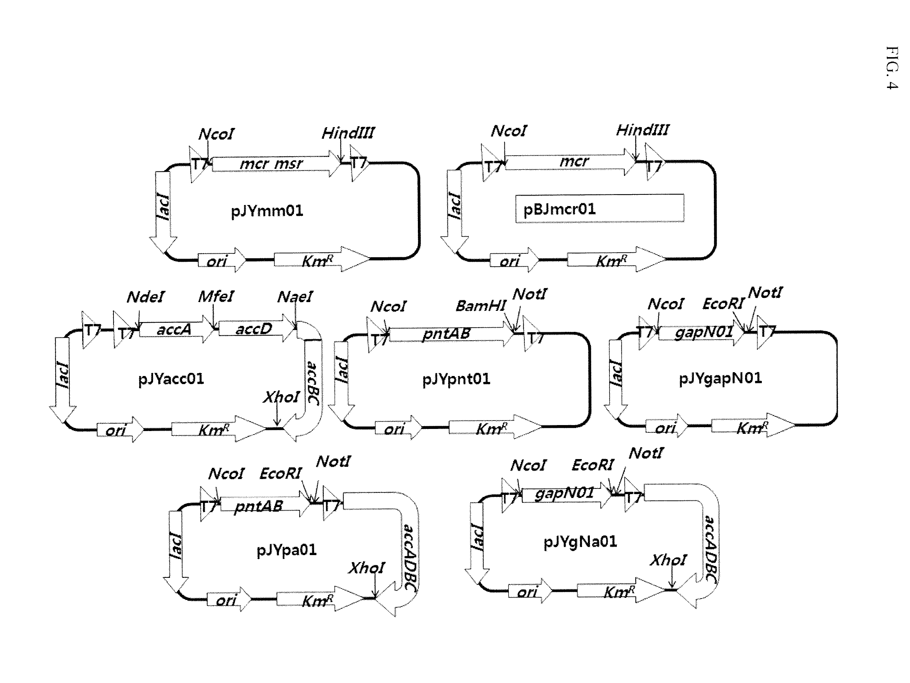

Therefore, in another aspect, the disclosure presents a recombinant vector containing one or more of a polynucleotide encoding an enzyme for reducing malonyl CoA into malonic semialdehyde and a polynucleotide encoding an enzyme for reducing malonic semialdehyde into 3-HP. The recombinant vector may also include a polynucleotide encoding a NADPH regeneration enzyme, e.g., *pnt*AB and/or *gap*N. Schematic maps of exemplary recombinant vectors are shown in FIG. 4.

Microbial Host

A recombinant vector according to an exemplary embodiment may be transformed into a suitable microbial host cell by conventional methods. A microbial host for producing 3-HP may be selected from bacteria, cyanobacteria, molds, and yeasts.

The microbial hosts selected for the production of 3-HP are tolerant to 3-HP and should be able to convert carbohydrates to 3-HP. The criteria for selection of suitable microbial hosts include the following: intrinsic tolerance to 3-HP, high rate of glucose utilization, availability of genetic tools for gene manipulation, and the ability to generate stable chromosomal alterations

Suitable host strains with a tolerance for 3-HP may be identified by screening based on the intrinsic tolerance of the strain. The intrinsic tolerance of microbes to 3-HP may be measured by determining the concentration of 3-HP that is responsible for 50% inhibition of the growth rate (IC₅₀) when grown in a minimal medium. The IC₅₀ values may be determined using methods known in the art. For example, the microbes of interest may be grown in the presence of various amounts of 3-HP and the growth rate monitored by measuring the optical density at 600 nanometers. The doubling time may be calculated from the logarithmic part of the growth curve and used as a measure of the growth rate. The concentration of 3-HP that produces 50% inhibition of growth may be determined from a graph of the percent inhibition of growth versus the 3-HP concentration. The host strain should have an IC₅₀ for 3-HP of greater than about 0.5%.

The microbial host for 3-HP production should also utilize glucose at a high rate. Most microbes are capable of utilizing carbohydrates. However, certain environmental microbes cannot utilize carbohydrates to high efficiency, and therefore would not be suitable hosts

A host also needs the ability to be transformed with exogenous nucleic acid to generate a recombinant microorganism. Gene transfer may be accomplished by a technique known in the art, such as electroporation, conjugation, transduction, or natural transformation. A conjugative plasmid with a broad host range and a drug resistance marker may be used. A cloning vector may be adjusted to a host based on properties of an antibiotic-resistance marker which can function in the host.

Furthermore, a microorganism host may also be manipulated to inactivate a competitive pathway for carbon flow by deletion or inactivation of various genes, or another method known in the art. For example, in the microorganism host a gene can be inactivated by direct inactivation or the availability of a transposon in a chromosome-integrated vector. In addition, a production host is subjected to chemical mutagenesis to obtain a mutant which improves innate isobutanol resistance of the host.

Based on the criteria described above, a suitable microbial host for producing 3-HP may include, but is not limited to, at least one microorganism from a genus selected from *Zymomonas, Escherichia*, *Pseudomonas*, *Alcaligenes*, *Salmonella*, *Shigella*, *Burkholderia*, *Oligotropha, Klebsiella*, *Pichia*, *Candida*), *Hansenula*, *Saccharomyces* and *Kluyveromyces.*

The host microorganism may be a microorganism from the genus *Escherichia,* the genus *Kluyveromyces*, or the genus *Sccharomyces.* For example, *Escherichia coli*, *Kluyveromyces marxianus*, *Kluyveromyces fragilis*, *Kluyveromyces lactis*, *Sccharomyces cerevisiae* may be used. More specifically, *Escherichia coli*, *Kluyveromyces marxianus*, or *Sccharomyces cerevisiae* may be used. Yet more specifically, in an exemplary embodiment, *E*. *coli* is used.

Among the possible host microorganisms (specifically, *E. coli*), some have multiple pathways to form acetate, and the formation of acetate is mediated from pyruvate by a pyruvate oxygenase ("POXB"), or from acetyl-CoA by a phophotransacetylase ("PTA") and an acetyl phosphate kinase ("ACKA").

Therefore, in an embodiment, the recombinant microorganism may include a microorganism from which one, or more, of the following genes is deleted or inactivated *adh*E, *pos*B, *pta*, *ack*, *frd* and/or *ldh*A gene.

### Construction of Recombinant Microorganism

A recombinant organism including the necessary genes that will encode the enzymatic pathway for conversion of a fermentable carbon substrate into 3-HP may be constructed using techniques known in the art. The genes may be transformed into a microorganism independently or simultaneously or inserted on a chromosome of the recombinant microorganism using one or more expression vectors.

In an embodiment, a gene encoding one of enzymes used in the 3-HP biosynthetic pathway, for example, a malonyl-CoA reductase, a malonic semialdehyde reductase, a pyridine nucleotide transhydrogenase or a glyceraldehyde-3-phosphate dehydrogenase, may be isolated from various sources, which are the same as those described above.

Methods of obtaining a desired gene from a bacterial genome are common and well known in the art of molecular biology. For example, when the sequence of a gene is known, suitable genomic libraries may be generated by restriction endonuclease digestion, and may be screened with probes complementary to the desired gene sequence. Once the sequence is isolated, the DNA may be amplified using standard primer-induced amplification methods, such as polymerase chain reaction ("PCR"), to obtain amounts of DNA suitable for transformation of a host using an appropriate vector. A codon optimizing tool is easily used to determine a variant sequence of the gene for optimizing expression of the gene in a heterologous host. Some codon optimizing tools may be used based on the GC content of a host organism. Methods for generating a desired codon-optimized variant polynucleotide sequence are known in the art.

Once a desired pathway gene is identified and isolated, the gene may transform a suitable expression host by a means known in the art. Transformation, transduction, or transfection may be achieved by any one of various means including, but not limited to, electroporation, microinjection, biolistics (or particle bombardment-mediated delivery), or agrobacterium-mediated transformation.

A vector or cassette useful in transformation of various host cells is common. Typically, the vector or cassette includes sequences directing transcription and translation of the desired gene, a selectable marker, and sequences allowing autonomous replication or chromosomal integration. Suitable vectors include a 5' region of the gene which harbors transcriptional initiation controls, and a 3' region of the gene which controls transcriptional termination. It should be understood that both control regions may be derived from genes homologous to the transformed host cell, but the control regions need not be derived from genes native to the species selected as the 3-HP production host.

Initiation control regions or promoters which are useful to drive expression of a coding region of a desired pathway gene in a desired host cell are numerous and familiar to those skilled in the art. Substantially, any promoters capable of driving these genes are suitable, and include, but are not limited to, CYC1, HIS3, GAL1, GAL10, ADH1, PGK, PHO5, GAPDH, ADCI, TRP1, URA3, LEU2, ENO, TPI (useful for expression in *Saccharamyces*); and lac, ara, tet, trp, 1PL, 1PR, T7, tac, and trc (useful for expression *in E. coli, Alcaligenes* and *Pseudomonas*) promoters.

In addition, termination control regions may also be derived from various genes native to the hosts. Optionally, a termination site may not be included, but can be included.

The terms "recombinant microorganism" and "recombinant host cell" are used interchangeably herein, and refer to microorganisms that have been genetically modified to overexpress or reduce expression of endogenous polynucleotides, or to express non-endogenous polynucleotide sequences, such as those included in a vector. The polynucleotide generally encodes an enzyme involved in a metabolic pathway for producing a desired metabolite as described above. Therefore, recombinant microorganisms described herein have been genetically engineered to express or overexpress target enzymes not previously expressed or overexpressed by the parent microorganism. It is understood that the terms "recombinant microorganism" and "recombinant host cell" refer not only to the specific original "recombinant microorganism but to the progeny or potential progeny of such a microorganism.

Also, it will be understood that not all vectors and expression control sequences function equally in expressing a DNA sequence of the invention disclosed herein. Likewise, not all hosts function equally for an identical expression system. However, those skilled in the art are able to make a suitable selection from various vectors, expression control sequences, and hosts, without departing from the scope of the invention. For example, a vector may be selected taking into consideration the selected host cell since the vector should be replicated in the host cell. The copy number of a vector, the ability to control the copy number, and expression of other proteins encoded by the vector, for example, an antibiotic marker, should be considered in selecting a suitable vector. Also, various vectors/ expression control sequences/host combinations suitable for use in the invention may be selected by those skilled in the art taking several factors into consideration.

In another example, a recombinant microorganism is provided. Deposited microorganisms are merely exemplary, and based on the inventive concept, a different species or genotype of an additional parent organism may be modified by one skilled in the art to realize the microorganism for producing 3-HP described herein.

### Method of Producing 3-HP

In still another aspect, a method of producing 3-HP is provided. In an embodiment, the method includes culturing the recombinant microorganism disclosed herein

In an embodiment, a method of producing 3-HP includes culturing the recombinant microorganism in a medium containing a carbon source collecting 3-HP the cultured microorganism. Here, culturing the recombinant microorganism and collecting the 3-HP may be performed by conventional methods known in the art forculturing a microorganism, and for isolation and purification of the 3-HPg.

As described above, in the 3-HP biosynthetic pathway, mcr and msr are involved in the pathway for biosynthesizing 3-HP in high yield from malonyl CoA via malonic semialdehyde. In addition, *pnt*AB *or gap*N increase the levels of NADPH and NADH in cells, thereby resolving redox imbalance associated with the engineered 3-HP biosynethetic pathway.

### Fermentation Medium

A fermentation medium must include a suitable carbon substrate. A suitable substrate may be, but is not limited to, a carbon source selected from the group consisting of a monosaccharide, an oligosaccharide, a polysaccharide, a C1 substrate, or a mixture thereof.

The substrate may be, but is not limited to, a monosaccharide such as glucose or fructose, an oligosaccharide such as lactose or sucrose, a polysaccharide such as starch or cellulose or a mixture thereof, and an unpurified mixture of renewable carbon source feedstocks. Additionally, the carbon substrate may be a C1 substrate such as carbon dioxide or methanol for which metabolic conversion into key biochemical intermediates has been demonstrated. In addition to C1 and C2 substrates, methylotrophics are known to utilize a number of other carbon-containing compounds such as various amino acids, glucosamine, and methylamine for metabolic activity. For example, methylotrophic yeast are known to utilize the carbon from methylamine to form trehalose or glycerol (Bellion et al., Microb. Growth C1 Compd., [Int. Symp.], 7th (1993), 415-32. (eds): Murrell, J. Collin Kelly, Don P. Publisher: Intercept, Andover, UK). Likewise, various species of Candida may metabolize alanine or oleic acid (Suiter et al., Arch. Microbiol. 153:485-489 (1990)). Therefore, the carbon source utilized herein may be selected from a variety of carbon-containing substrates, and o limited only by the metabolism of the microorganism.

Although it is considered that all of the above carbon substrates and mixtures thereof are suitable when they can create conditions under which the enzyme pathway disclosed herein can function, the carbon substrate used in an exemplary embodiment may be glucose, sucrose, cel lulose, or glycerol.

In addition to a suitable carbon source, the fermentation medium may include a suitable mineral, salt, enzymatic cofactor, buffer, and other components known to those skilled in the art which are suitable for stimulating the disclosed enzymatic pathway required to produce 3-HP and/or for growth of the cell culture.

### Culture Conditions

Typically, cells are grown at a temperature in the range of about 25 °C to about 40 °C in an appropriate medium. A suitable growth medium can be a commercially prepared medium such as Luria Bertani ("LB") broth, Sabouraud Dextrose ("SD") broth, or Yeast Medium ("YM") broth.

Other defined or synthetic growth medium may also be used, and the appropriate medium for the growth of the specific microorganism will be known by one skilled in the art of microbiology or fermentation science.

A suitable pH for fermentation is between about pH 5.0 to about pH 9.0. More specifically, the initial pH for culture and fermentation may be about pH 6.0 to about pH 8.0. Fermentation may be performed under aerobic or anaerobic conditions. In an embodiment, fermentation is performed under under an anaerobic or microaerobic condition.

### Industrial Batch and Continuous Fermentation

A batch fermentation method may be used. A classical batch fermentation is a closed system in which the composition of the medium is established at the beginning of the fermentation and not subjected to artificial alterations during the fermentation. Therefore, at the beginning of the fermentation, the medium is inoculated with the desired organism, and the fermentation is permitted to occur without adding anything to the system. However, often, "batch" fermentation is a batch-type fermentation with respect to the addition of the carbon source, while attempts are often made at controlling factors such as pH and oxygen concentration during the fermentation. In batch systems, the metabolite and biomass composition of the system change constantly up to the time the fermentation is stopped. Within batch cultures, cell growth proceeds through a static lag phase to an exponential growth (log) phase and finally to a stationary phase where the growth rate is decreased or stopped. Intact cells in the stationary phase will eventually die. Generally, cells in the log phase are involved in bulk-formation of final products and/or intermediates.

A modified version of the standard batch process is a fed-batch process. A fed-batch fermentation processis a typical batch process except that a growth-limiting substrate is incrementally added as the fermentation progresses. A fed-batch process is useful in various circumstances, such as when catabolite repression is apt to inhibit metabolic action in the cells or where it is desirable to have limited amounts of substrate in the medium, for example to control the reaction rate. In a fed-batch system, the actual substrate concentration is difficult to measure, and is therefore calculated based on the change in measurable factors such as pH, dissolved oxygen, or the partial pressure of a waste gas, such as CO₂. Batch and fed-batch fermentations are common and well known in the art (Thomas D. Brock in Biotechnology: A Textbook of Industrial Microbiology, Second Edition (1989) Sinauer Associates, Inc., Sunderland, MA or Desphande, Mukund (Appl. Biochem. Biotechnol. 36(3):227-234 (1992)).

Production of 3-HP is considered possible not only by batch fermentation, but also by continuous fermentation. A continuous fermentation is an open system in which a defined fermentation medium is added continuously to the bioreactor and an equal amount of conditioned medium is removed simultaneously for processing. Continuous fermentation permits cells to be maintained at a constant high density where the cells are mainly in log-phase growth.

Continuous fermentation allows for the modulation of one factor or any number of factors that affect cell growth or final product production. For example, one method will maintain a limiting nutrient such as the carbon source or nitrogen level at a fixed concentration and allow all other parameters to moderate. In other systems, a number of factors affecting growth can be altered continuously while the cell concentration, measured by medium turbidity, is kept constant. Continuous systems strive to maintain steady-state growth conditions and thus the cell loss due to the medium being drawn off must be balanced against the cell growth rate during the fermentation. Methods of modulating nutrients and growth factors for continuous fermentation processes, as well as techniques for maximizing the rate of cell formation, are well known in the art of industrial microbiology and a variety of methods are detailed in Brock's textbook.

Exemplary embodiments may be performed using any one of the batch, fed-batch, and continuous processes.

The maximum yield of 3-HP produced using the recombinant microorganism disclosed herein by the method as described above is about 97% (w/w) or more. For example, 97% mol 3-HP may be produced per mol.

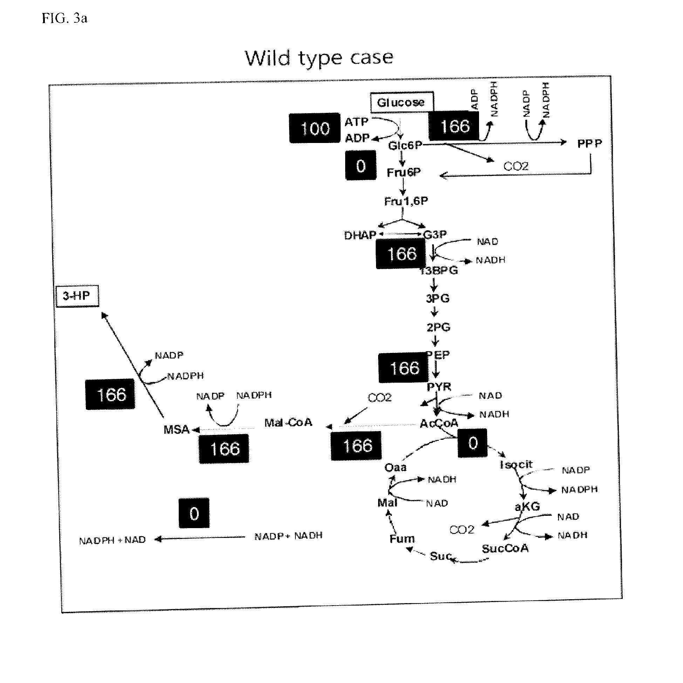

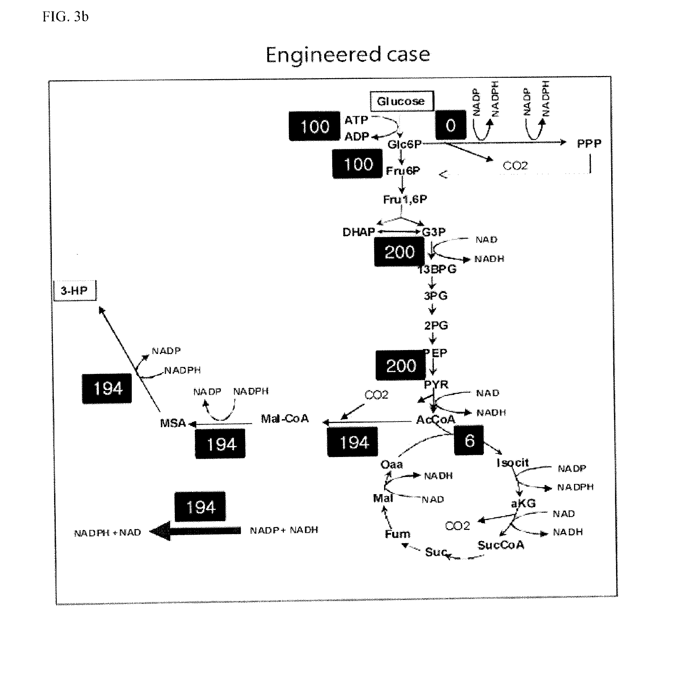

A theoretical yield from glucose to 3-HP in the wild-type microorganism and the recombinant microorganism is shown in Fig. 3A and 3B, respectively. The theoretical yield in the wild-type microorganism is 0.83% (166 mol 3-HP/100 mol glucose), whereas the theoretical yield in the recombinant microorganism is 0.97% (194 mol 3-HP/100 mol glucose). Therefore, the productivity of 3-HP is drastically increased, which makes it possible to mass-produce 3-HP in an industrial scale.

As a result of metabolic engineering, the genetic recombinant microorganism disclosed herein has considerably increased in productivity of 3-HP by using a different metabolic pathway from the conventional one in the parent microorganism and by resolving the redox imbalance accompanying the introduction of the new 3-HP biosynthetic path.

Hereinafter, an embodiment of the invention will be described in further detail with respect to Examples. These Examples merely describe the embodiment in detail, and the invention is not limited to these Examples.

Particularly, while, in the following Examples, a specific expression vector and *E*. *coli* host cells are exemplified to express a gene according to the invention, it is clearly understood by those skilled in the art that various kinds of expression vectors and host cells are also used.

### General Methods

Procedures for cloning a standard recombinant DNA and molecules used in the Examples are known in the art. Techniques suitable for use in the following examples may be found in Sambrook et al. [Sambrook, J., Fritsch, E. F. and Maniatis, T., Molecular Cloning: A Laboratory Manual; Cold Spring Harbor Laboratory Press, Cold Spring Harbor, 1989], Silhavy et al. [Silhavy et al., Experiments with Gene Fusions, Cold Spring Harbor Laboratory, Cold Spring Harbor, N.Y. 1984], and Ausubel et al. [Ausubel et al., Current Protocols in Molecular Biology, pub. by Greene Publishing Assoc. and Wiley-Interscience, 1987].
Materials and methods suitable for maintenance and growth of a bacterial culture are known in the art. Suitable techniques to be used in the following Examples can be seen in the following: Manual of Methods for General Bacteriology, Phillipp et al., eds., American Society for Microbiology, Washington, DC.,1994 and Thomas D. Brock, Biotechnology: A Textbook of Industrial Microbiology, Second Edition, Sinauer Associates, Inc., Sunderland, MA (1989).

Unless described otherwise, all reagents, restriction enzymes, and materials used to grow and maintain bacterial cells are obtained from Sigma Chemical Company (St. Louis, MO., USA).

### Example 1. Gene Cloning

(1) Cloning of *M. sedula-derived* mcr and msr Genes

Polynucleotides for each of the *M sedula* mcr and msr ("mm") genes were synthesized by Bioneer (KR) with a codon-optimized sequence to permit optimal enzyme expression in *E. coli.*

(2) Cloning of *E*. *coli-derived pntAB* and *S*. *mutants-derived gapN* Genes

An *E. coli pnt*AB polynucleotide sequence was obtained by E. coli genome polymerase chain reaction (PCR) (primers: 5'-AATT CCA TGG GA CGA ATT GGC ATA CCA AGA GAA C and 5'-AATT GGA TTC TTA CAG AGC TTT CAG GAT TGC ATC). A polynucleotide for the *S*. *mutants gap*N gene was synthesized by Bioneer (KR) with a codon-optimized sequence for expression in *E. coli.*

### Example 2. Construction of Recombinant Vectors

**A. pJYmm01**

The expression vector pCDFDuet-1 (EMD Chemicals) was used to construct a recombinant vector for expressing the polynucleotide with the codon-optimized mcr and msr ("mm") genes. The pCDFDuet-1 was digested with NcoI and HindIII restriction enzymes, and an mm sequence, digested with the same restriction enzymes, was ligated into the vector, thereby obtaining a plasmid designated pJYmm01.

**B. pBJmcr01**

The expression vector pCDFDuet-1 (EMD Chemicals) was used to construct a recombinant vector for expressing the mcr gene of *Chloroflexus aurantiacus.* The *Chloroflexus aurantiacus* mcr gene was obtained by *C*. *aurantiacus* genome PCR. The mer gene amplicon was digested with NcoI and HindIII restriction enzymes, and then ligated with pCDFDuet-1, digested with the same restriction renzymesto obtain the plasmid designated pBJYmcr01.

**C. pJYacc01**

The expression vector, pRSFDuet-1 (EMD Chemicals), was used to construct a recombinant vector expressing the E. coli acetyl CoA carboxylase. A polynucleotide of the acetyl CoA carboxylase A ("*acc*A") gene was obtained by *E. coli* genome PCR. The *accA* polynucleotide sequence was digested with NdeI and MfeI restriction enzymes, and then ligated into pRSFDuet-1 prepared by the same restriction digestion method, thereby obtaining the plasmid designated pJYaccA.

Further, an *acc*D polynucleotide sequence was obtained by *E. coli* genome PCR, and then digested with MfeI and NaeI restriction enzymes. The restricted *acc*D sequence was then ligated into pJYaccA, digested with the same restriction enzymes to obtain the plasmid designated pJYaccAD.

Similarly, an *acc*BC polynucleotide sequence was obtained by *E. coli* genome PCR, and digested with NaeI and XhoI restriction enzymes. The resulting *acc*BC sequence was ligated into pJYaccAD digested with the same restriction enzymes to obtain the plasmid designated pJYace01.

**D. pJYpnt01**

The expression vector, pRSFDuet-1 (EMD Chemicals) was used to construct a recombinant vector expressing *E. coli pnt*AB*.* The *pnt*AB polynucleotide sequence was obtained by *E*. *coli* genome PCR. The *pnt*AB polynucleotide was digested with Ncol and BamHI restriction enzymes, and then ligated into pRSFDuet-1 prepared by the same restriction digestion methodto obtain the plasmid designated pJYpnt01.

**E. pJYgapN01**

The expression vector, pRSFDuet-1 (EMD Chemicals) was used to construct a recombinant vector to express the *S*. *mutants gap*N gene., and *gapN* used a codon-optimized sequence (Bioneer, Korea) to be expressed in *E. coli.* The pRSFDuet-1 and the codon-optimized *gap*N polynuceotide sequence were each digested with Ncol and EcoRI restriction enzymes. The two polynucleotides were then ligated together, thereby obtaining the plasmid pJYgapN01.

**F. pJYpa01**

The plasmid pJYacc01 obtained in **B** was digested with NotI and XhoI restriction enzymes to obtain the *acc* sequence. The *acc* sequence was ligated into pJYpnt01 partially digested by the same restriction enzymes, thereby obtaining the plasmid pJYpa01.

**G. pJYgNa01**

The pJYacc01 obtained in **B** was digested with NotI and XhoI restriction enzymes to obtain the *acc* sequence. The *acc* sequence was ligated with pJYgapN01 partially digested by the same restriction enzymes, thereby obtaining the plasmid pJYgNa01.

Maps of each of the recombinant vectors disclosed herein are illustrated in FIG. 4:
(1) a vector containing the mcr-msr (*M*. *sedula*) genes;
(2) a vector containing an mcr (*C*. *aurantiacus*) gene;
(3) a vector containing an *acc* gene;
(4) a vector containing a *pnt*AB gene;
(5) a vector containing a *gap*N gene;
(6) a vector containing a *pnt*AB-*acc* gene; and
(7) a vector containing a *gap*N-*acc* gene.

### Example 3. Preparation of Recombinant E.coli for producing 3-HP

(1) Recombinant Strain

The recombinant vectors constructed in Example 2 were introduced into *E. coli* to prepare a number of recombinant strains. The vectors manufactured above were transformed into *E*. *coli* by electroporation.

(2) Measurement of mcr Enzymatic Activity

In Journal of Bacteriology, Dec 2006, vol. 188, no. 24, p8551-8559 (p8552), when NADPH was used as a substrate for the mcr, the change in amount of NADPH into NADP due to the enzyme reaction was analyzed by a spectrophotometric assay.

A cell extract or purified enzyme was used. A predetermined amount of NADPH was added to a buffer, and the prepared cell extract or enzyme was added. The resulting cells were incubated at 65 °C for 5 minutes. Malonyl-CoA was added to start the reaction, and absorbance at 365 nm (A₃₆₅) of samples before and after the reaction were measured. 1 unit of mcr enzyme represents the amount of the mcr enzyme oxidizing 1 micromole of NADPH per minute (the same as the amount of the mcr enzymereducing 1 micromole of malonyl CoA per minute).

Expression of the genes in other host microorganisms would require a procedure analogous to that presented here.

### Example 4. Production of 3-HP using Transformed E. coli

The recombinant strains contstructed in Example 3 were incubated in 2L M9 medium containing 100 mM potassium phosphate buffer (pH 7.0), 20 g/L glucose and suitable antibiotics (50 µg/ml streptomycin and 50 µg/ml kanamycin) in a 5-L bioreactor (KO biotech, Seoul, Korea). The recombinant strains obtained in overnight culture were inoculated into a medium with OD₆₀₀ of 0.2.

While glucose (15 g/L) was sporadically added, aerobic fermentation was performed in a fed-batch mode. The temperature and rotation speed were maintained at 37 °C and 500 rpm, respectively. During the fermentation, the flow of sterile air was constantly maintained at an aeration rate of 0.35vvm. Unless described otherwise, pH was maintained at 7.0 with 5N NaOH and 5N HCl.

Recombinant cells were introduced into 0.1 mM IPTG with OD₆₀₀ of 1.3, and 0.1 mM IPTG was added thereto every 12 hours. A medium composition containing antibiotics was added every 12 hours.

Concentrations of glucose and 3-HP were measured by HPLC using a refractive index ("RI").

A sample incubated at 10000 x g for 5 minutes was centrifuged. The obtained supernatant was filtered using 0.2 mM Tuffryn-membranes and separated with a 300 mm x 7.8 mm Aminex HPX-87H (Bio-Rad, USA) column using 5.41 mM H₂SO₄ at 50 °C. The flow rate was 0.6 ml/min.

(1) When Transformed with mcr-msr Gene

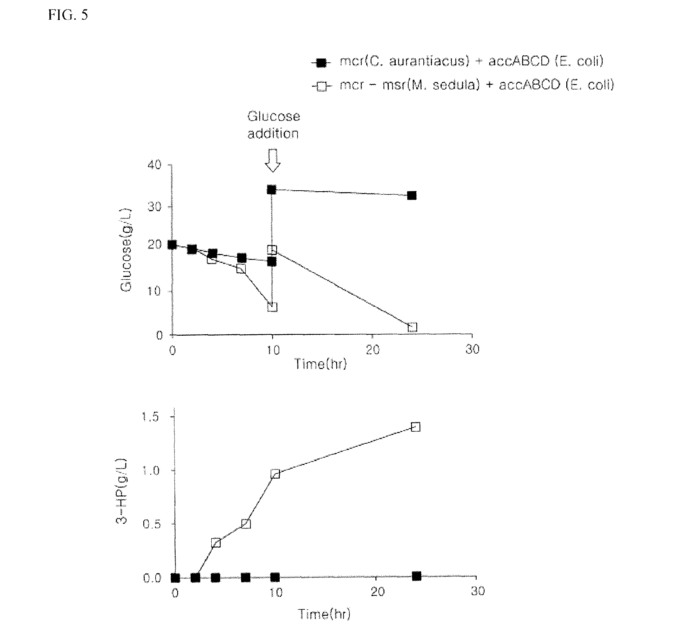

The result is shown in FIG. 5.

It can be confirmed that the most effective pathway for conversion of malonyl CoA into 3-HP based on enzyme activity was a "malonic semialdehyde reducing pathway" reducing acetyl CoA into 3-HP in an order of acetyl CoA, malonyl CoA, malonic semialdehyde and 3-HP.

(2) When Transformed with *mcr-msr-pnt*AB Gene or *mcr-msr-gapn*N Gene

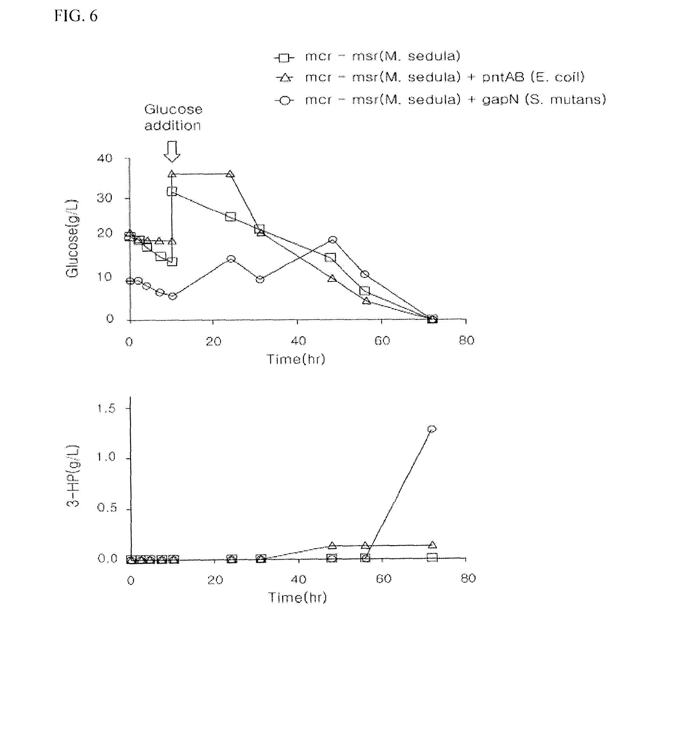

The result is shown in FIG. 6 and Table 1.

**Table 1. Glucose (g/L) present in fermentation**

| time | *mcr-msr (M. sedula)* | *mcr-msr (M. sedula)*⁺ *pnt*AB (*E. coli*) | *mcr-msr (M. sedula)* + *gap*N (*S. mutants*) |
|---|---|---|---|
| 0 | 20.65464 | 21.5028 | 9.94302 |
| 2 | 19.84248 | 20 | 9.80136 |
| 4 | 17.94897 | 19.8 | 8.57529 |
| 7 | 15.88941 | 19.7 | 7.03467 |
| 10 | 14.43681 | 19.42569 | 6 |
| 10 | 31.73841 | 36 | 15.26364 |
| 24 | 25.45605 | 35.80884 | 11.62908 |
| 31 | 22.33521 | 21.94893 | 10.42461 |
| 31 | 22.33521 | 21.94893 | 19.72467 |
| 48 | 15.28587 | 10.2969 | 11.3103 |
| 56 | 7 | 5 | 5 |
| 72 | 0 | 0 | 0 |

Referring to Table 1, it can be confirmed that when an NADPH regeneration enzyme was introduced into cells to increase NADPH and NADH levels, the redox imbalance was resolved, and thus the 3-HP yield was considerably increased (see FIG. 5).

Particularly, compared to when the cell was transformed with a *pnt*AB gene (coding for a pyridine nucleotide transhydrogenase), when the cell was transformed with a *gap*N gene (coding for a glyceraldehyde-3-phosphate dehydrogenase), that is, when the transformation was performed with the activity of the glyceraldehyde-3-phosphate dehydrogenase, the capability of producing 3-HP was further increased (see the bottom graph of FIG. 6).

Therefore, it can be seen from the results that when the "malonic semi aldehyde reducing pathway" is used with the NADPH regeneration enzymes, for example, *gap*N or *pnt*AB and *gap*N, 3-HP available in an industrial scale may be produced with a high yield.

While exemplary embodiments have been disclosed herein, it should be understood that other variations may be possible. Such variations are not to be regarded as a departure from the scope of exemplary embodiments of the present application, and all such modifications as would be obvious to one skilled in the art are intended to be included within the scope of the following claims.

## Claims

1. A recombinant microorganism comprising
an enzyme for reducing malonyl CoA into malonic semialdehyde, and
an enzyme for reducing malonic semialdehyde into 3-hydroxypropionic acid (3-HP), such that the recombinant microorganism is capable of producing 3-HP from Acetyl CoA.

2. The recombinant microorganism of claim 1, wherein the enzyme for reducing malonyl CoA into malonic semialdehyde has a malonyl CoA reductase ("mcr") activity, and the enzyme of reducing malonic semialdehyde into 3-HP has a malonic semialdehyde reductase ("msr") activity.

3. The recombinant microorganism of claim 2, wherein the mcr enzyme and msr enzyme are exogenous.

4. The recombinant microorganism of claim 3, wherein the mcr enzyme and the msr enzyme are derived from *M. sedula*.

5. The recombinant microorganism of claim 1, further comprising an NADPH regeneration activity.

6. The recombinant microorganism of claim 5, wherein the NADPH regeneration activity is a transhydrogenase activity or a glyceraldehyde-3-phosphate dehydrogenase activity.

7. The recombinant microorganism of claim 6, wherein the transhydrogenase activity is a pyridine nucleotide transhydrogenase ("*pnt*AB") activity or a soluble pyridine nucleotide transhydrogenase ("*udh*A") activity.

8. The recombinant microorganism of claim 7, wherein the *pnt*AB activity is derived from *E*. *coli.*

9. The recombinant microorganism of claim 6, wherein the glyceraldehyde-3-phosphate dehydrogenase activity is a *gapN* activity, and the *gapN* activity is derived from S. *mutants.*

10. The recombinant microorganism of claim 1, wherein the recombinant microorganism is at least one selected from the group consisting of genus *Zymomonas*, genus *Escherichia*, genus *Pseudomonas*, genus *Alcaligenes*, genus *Salmonella*, genus *Shigella*, genus *Burkholderia*, genus *Oligotropha*, genus *Klebsiella*, genus *Pichia*, genus *Candida*, genus *Hansenula*, genus *Saccharomyces* and genus *Kluyveromyces.*

11. The recombinant microorganism of claim 10, wherein the recombinant microorganism is *Escherichia coli*, *Kluyveromyces marxianus or Sccharomyces cerevisiae.*

12. The recombinant microorganism of claim 10, wherein an *adh*E, *pos*B, *pta*, *ack*, *frd* and/or *ldh*A gene is deleted in the recombinant microorganism.

13. A recombinant expression vector which is at least one selected from the group consisting of
a vector comprising an mcr-msr gene,
a vector comprising an mcr-msr gene and *pnt*AB genes,
a vector comprising an mcr-msr gene and a *gap*N gene, and
a vector comprising an mcr-msr gene, *pnt*AB genes, and *agap*N gene.

14. A method of producing 3-hydroxypropionic acid (3-HP), the method comprising:
culturing the recombinant microorganism of claim 1 under conditions such that 3-hydroxypropionic acid (3-HP) is produced.

15. The method of claim 14, wherein the microorganism is cultured on a medium comprising at least one carbon substrate selected from the group consisting of glucose, sucrose, cellulose and glycerol.

16. The method of claim 14, further comprising isolating the 3-HP.


SEQUENCE LISTING

<110> SAMSUNG ELECTRONICS CO., LTD.

<120> METHOD OF PRODUCING 3-HYDROXYPROPIONIC ACID USING MALONIC
SEMIALDEHYDE REDUCING PATHWAY

<130> EP79985FZ200pau

<140> 12160658.6
<141> 2012-03-22

<150> 10-2011-0026531
<151> 2011-03-24

<160> 2

<170> PatentIn version 3.2

<210> 1
<211> 34
<212> DNA
<213> artificial sequence

<220>
<223> PCR primer

<400> 1
aattccatgg gacgaattgg cataccaaga gaac 34

<210> 2
<211> 34
<212> DNA
<213> artificial sequence

<220>
<223> PCR primer

<400> 2
aattggattc ttacagagct ttcaggattg catc 34